# EUROPEAN PATENT APPLICATION

(11) **EP 4 616 869 A1**
(43) Date of publication of application: **17.09.2025**
(21) Application number: 24382290.5
(22) Date of filing: 15.03.2024
(51) Int. Cl.: A61K 47/68, A61P 35/00, C07K 16/40

(54) **FAP TARGETED ANTIBODY-DRUG CONJUGATES**

(71) Applicant: Oncomatryx Biopharma, S.L., 48160 Derio (Vizcaya) (ES)
(72) Inventor: EGANA GANUZA, Isabel, DERIO, BIZKAIA (ES); FABRE, Myriam, DERIO, BIZKAIA (ES); SIMON BUELA, Laureano, DERIO, BIZKAIA (ES)
(74) Representative: Mewburn Ellis LLP

(57) **Abstract**

The present invention relates to anti-FAP-targeted antibody-drug conjugates (ADCs), in particular anti-FAP antibody-drug conjugates having the formula A-(L-D)p, or a pharmaceutically acceptable salt or solvate thereof, wherein A is an anti-FAP antibody, L is a linker, D is a drug comprising a dolastatin and p is 1 to 10. Methods of using such conjugates in the treatment of cancer are also disclosed.

## Description

### Field of the Invention

The present invention relates to antibody-drug conjugates (ADCs) that target fibroblast activation protein, and to their use in medicine, e.g. in the treatment of cancer.

### Background

Antibody-drug conjugates (ADCs) are a rapidly growing class of biopharmaceutical drugs intended as directed therapies for the treatment of cancer. These are highly targeted molecules that combine the specificity of surface antigen-specific monoclonal antibodies (mAbs) with the highly potent cytotoxicity of effector small chemical molecules. Their purpose is to induce target cell death, thereby attacking and killing tumor cells while sparing healthy cells. The primary mechanism of action of ADCs involves the specific binding of the monoclonal antibody component to target antigens on the surface of cancer cells. Upon binding, the ADC is internalized by cancer cells, resulting in intracellular release of cytotoxic payload and ultimately cell death.

ADCs have emerged as a powerful class of therapeutic drugs and show promising effect in the treatment of various cancer types. They have the potential to improve therapeutic efficacy and reduce systemic toxicity that often occurs with small molecule drugs. They are designed to maximize antitumor activity while minimizing damage on normal tissue, potentially improving the therapeutic index. A variety of ADCs have been approved by the FDA, and nearly 100 ADCs are currently under development, undergoing preclinical and clinical studies (Shastry, Mythili *et al*.; Fu, Zhiwen *et al*.; Pettinato MC).

Fibroblast Activating Protein (FAP) is a 170 kDa membrane-bound gelatinase belonging to the S9B prolyl oligopeptidase subfamily. This type II transmembrane glycoprotein, with enzymatic activities including dipeptidase and endopeptidase functions, participates in various biological processes such as pericellular proteolysis of the extracellular matrix, endothelial cell migration and invasion. It plays a role in tissue remodelling and is highly expressed in tumor stroma, making it a potential target for cancer therapy. Oncomatryx developed OMTX705, an ADC for the treatment of solid tumors that contains a monoclonal antibody targeting the FAP glycoprotein, conjugated to a cytolysin payload. The ADC has shown promising results in preclinical studies and is currently being evaluated in clinical trials for its effectiveness against various types of late-stage solid tumors.

Cytolysins are a class of synthetic tetrapeptide analogs of natural tubulysins, which have low molecular weight and show high cytotoxic activity. They are not substrates of Pgp and thus do not generate multidrug resistance. They exert their mechanism of action by binding to tubulin to inhibit its polymerization and prevent microtubule depolymerization. This results in the disruption of microtubules, leading to disassembly and cell cycle arrest. Therefore, cytolysins are used as payloads of ADCs, such as OMTX705, due to their potent cytotoxic effects.

There are many possible payloads for ADCs. These can generally be categorised based on their mechanism of action, for example tubulin inhibitors, DNA damaging agents and immunomodulators (see *e.g.* Fu et al. Nature (2022) 7(1):93). When it comes to ADC design, there are many options for targets, payloads and linkers, and each component of the ADC must be compatible to achieve high specificity, efficacy and safety (see *e.g.* Marei et al. Cancer Cell International (2022) 22(1):255). One example of a cytotoxic payload used in ADCs is the dolastatin 10 derivative, monomethyl auristatin E.

Monomethyl auristatin E (MMAE) is another potent cytotoxic agent commonly used as payload of antibody-drug conjugates (ADCs). It inhibits cell division by blocking tubulin polymerization and exerts cytotoxic effects by binding to microtubules and preventing cell proliferation. MMAE meets key requirements for an ideal ADC payload, including high cytotoxicity, well-defined target and mechanism of action, potential chemical binding sites, high stability during circulation, uptake and release into the target, and good solubility. Studies have shown that MMAE is a substrate of multiple drug resistance and is therefore secreted by MDR1 and excreted from cells. This MDR1-mediated efflux mechanism may influence the cytotoxic effects of antibody-drug conjugates (ADCs) containing MMAE payloads. This payload is used in FDA-approved ADCs such as Adcetris^{®} and Polivy^{®} and has shown potent activity in clinical trials for various cancers, including lymphoma, leukemia, lung, gastric, prostate, and breast cancer.

While both MMAE and cytolysin are used as payloads in ADCs for cancer therapy, they differ in their mechanisms of action, interactions with drug resistance proteins, and potential implications for toxicity and safety of the corresponding ADCs (Wang, Zhijia *et al*.) (Samantasinghar, Anupama *et al*.) (Riccardi, Federico *et al*.)*.*

Zana *et al.* (2022) describes FAP triggering the release of drug payload from non-internalising small molecule drug conjugates in solid tumours (see Zana et al. Clin Cancer Res. (2022) 28(24):5440-5454). Nadal *et al.* describes the generation and *in vivo* validation of an IL-12 fusion protein based on a novel anti-human FAP monoclonal antibody (see Nadal et al., J Immunother Cancer. (2022) 10(9):e005282). Zana *et al.* (2023) discloses a comparative analysis of FAP-targeted small molecule-drug, antibody-drug, and peptide-drug conjugates (see Zana et al. Bioconjug Chem. (2023) 24(7):1205-1211).

The present invention has been devised in light of the above considerations.

### Summary of the Invention

Broadly, the present invention relates to anti-FAP antibodies, conjugates thereof and payloads for use in antibody-conjugate strategies. In particular, the present inventors have found that the anti-FAP ADCs described herein exhibit selective binding to the extracellular region of FAP, efficient internalisation, and surprising *in vitro* and *in vivo* efficacy.

Accordingly, in a first aspect, the present invention provides a conjugate having the formula I:

(I) A-(L-D)ₚ

or a pharmaceutically acceptable salt or solvent thereof,
wherein:
A is an antibody or antigen-binding fragment thereof comprising
   (i) a heavy chain variable (VH) region comprising the following CDRs:
      HC-CDR1 having the amino acid sequence of SEQ ID NO:7
      HC-CDR2 having the amino acid sequence of SEQ ID NO:8
      HC-CDR3 having the amino acid sequence of SEQ ID NO:9
      or a variant thereof having one amino acid variation in one or more of HC-CDR1 , HC-CDR2, or HC-CDR3; and
   (ii) a light chain variable (VL) region comprising the following CDRs:
      LC-CDR1 having the amino acid sequence of SEQ ID NO:10
      LC-CDR2 having the amino acid sequence of SEQ ID NO:11
      LC-CDR3 having the amino acid sequence of SEQ ID NO:12
      or a variant thereof having one amino acid variation in one or more of LC-CDR1, LC-CDR2, or LC-CDR3;
L is a linker;
D is a drug comprising a dolastatin; and
p is 1 to 10.

In some embodiments of the first aspect, A comprises:
(i) a heavy chain variable (VH) region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9; and
(ii) a light chain variable (VL) region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12.

In some embodiments of the first aspect, A comprises a heavy chain variable (VH) region comprising an amino acid sequence having at least 90%, 95%, 99% or 100% sequence identity with SEQ ID NO:5. In some embodiments, A comprises a heavy chain variable (VH) region comprising the amino acid sequence of SEQ ID NO:5.

In some embodiments of the first aspect, A comprises a light chain variable (VL) region comprising an amino acid sequence having at least 90%, 95%, 99% or 100% sequence identity with SEQ ID NO:6. In some embodiments, A comprises a light chain variable (VL) region comprising the amino acid sequence of SEQ ID NO:6
In some embodiments of the first aspect, A comprises a heavy chain comprising an amino acid sequence having at least 90%, 95%, 99% or 100% sequence identity with SEQ ID NO:3. In some embodiments, A comprises a heavy chain comprising the amino acid sequence of SEQ ID NO:3.

In some embodiments of the first aspect, A comprises a light chain comprising an amino acid sequence having at least 90%, 95%, 99% or 100% sequence identity with SEQ ID NO:4. In some embodiments, A comprises a light chain comprising the amino acid sequence of SEQ ID NO:4.

In some embodiments of the first aspect, D comprises monomethyl auristatin E (MMAE) or monomethyl auristatin F (MMAF). In some embodiments, D comprises MMAE.

In some embodiments of the first aspect, L comprises a protease-cleavable linker, wherein the linker is cleavable by an intracellular protease, optionally wherein the intracellular protease is a cathepsin.

In some embodiments of the first aspect, L comprises a valine-citrulline unit. In some embodiments, L comprises p-aminobenzylcarbamate. In some embodiments, L comprises maleimidocaproyl-valine-citrulline-p-aminobenzylcarbamate.

In some embodiments of the first aspect, L comprises a spacer. In some embodiments, the spacer has a chain length of 2 to 30 atoms. In some embodiments, the spacer comprises or consists of an alkylene or oxyalkylene group. In some embodiments, the spacer comprises between one and six ethylene glycol units, e.g. a triethylene glycol.

In some embodiments of the first aspect, -L-D has the following structure: 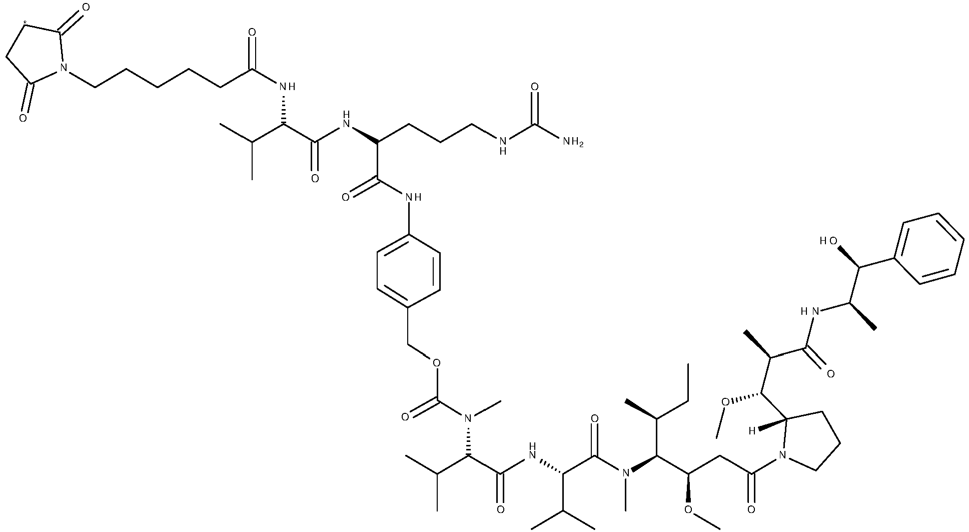 wherein * indicates the site of attachment to A.

In some embodiments of the first aspect, the conjugate is the conjugate described herein as OMTX105.

In some embodiments of the first aspect, p is 1, 2, 3, 4 or 5.

In a second aspect, the present invention provides a pharmaceutical composition comprising a conjugate according to the first aspect and a pharmaceutically acceptable carrier, excipient or diluent.

In some embodiments of the second aspect, the pharmaceutical composition comprises a plurality of conjugates according to the first aspect. In some embodiments, p is provided as an average of the plurality of conjugates. In some embodiments, when p is provided as an average of the plurality of conjugates, p is between 3 and 4. In some embodiments, p is between 3.5 and 4.

In a third aspect, the present invention provides a conjugate according to the first aspect, or a composition according to the second aspect, for use in medicine.

In a fourth aspect, the present invention provides a conjugate according to the first aspect, or a composition according to the second aspect, for use in a method of treatment of cancer in a mammalian subject.

In a fifth aspect, the present invention provides a method of treating cancer in a mammalian subject comprising administering a therapeutically effective amount of a conjugate according to the first aspect, or a composition according to the second aspect.

In a sixth aspect, the present invention provides the use of a conjugate according to the first aspect, or a composition according to the second aspect, in the manufacture of a medicament for use in a method of treating cancer in a mammalian subject.

In some embodiments of the fourth to the sixth aspects, the method comprises simultaneous, sequential, or separate administration of one or more other anti-cancer agents. In some embodiments, the one or more other anti-cancer agents comprise a cytotoxic chemotherapy agent, an anti-angiogenic agent or an immunotherapeutic agent. In some embodiments, the one or more other anti-cancer agents comprise anti-programmed cell death protein 1 (PD-1) antibodies or anti-programmed death-ligand 1 (PD-L1) antibodies. In some embodiments, the one or more other anti-cancer agents comprise Nivolumab (MDX1106) or Pembrolizumab (MK-3475).

In some embodiments of the third to the fifth aspects, the cancer comprises a solid tumor. The cancer may be pancreatic cancer, sarcoma (for example, fibrosarcoma), oesophageal cancer, breast cancer, melanoma, lung cancer, gastric cancer, head and neck cancer, ovarian cancer, bladder cancer or colorectal cancer. The cancer may be exocrine pancreatic cancer. In particular, the cancer may be pancreatic ductal adenocarcinoma.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Figure 1****.** *In vitro* comparative activity of MMAE and cytolysin A1 B1 payloads through cell viability assay in HCT15 and HCT116 cells. Cell viability (%) over a range of concentrations of each compound.
**Figure 2****.** FAP binding analysis of OMTX105 and OMTX705 ADCs through flow cytometry on HT1080-FAP and HT1080-WT cells. Relative MFI of bound OMTX705 and OMTX105 ADCs on HT1080FAP and HT1080WT cell lines.
**Figure 3****.** Cytotoxic activity of OMTX105 and OMTX705 ADCs through cell viability assay on FAP-expressing and parental HT1080 cells. Cell viability (%) over a range of protein concentration of both ADCs.
**Figure 4****.** Tumor growth and body weight change monitorization in NOD scid gamma (NSG) mice subcutaneously injected with HT1080-FAP cells and following weekly intravenous administration of control Vehicle, OMTX10520 mg/kg and OMTX705 20 mg/kg. (A) Tumor volume (mm3) over time. (B). Relative animal body weight change over time.
**Figure 5****.** Tumor growth monitorization in Panc007 pancreatic ductal adenocarcinoma tumor patient-derived xenograft mice upon OMTX105 and OMTX705 treatment. Tumor volume (mm3) over time following weekly administration of control Vehicle, OMTX105 (5 and 20 mg/kg) and OMTX705 (5 and 20 mg/kg).
**Figure 6****.** Body weight changes upon treatment with anti-FAP OMTX105 and OMTX705 ADCs in Panc007 pancreatic ductal adenocarcinoma tumor patient-derived xenograft mice. Relative murine body weight change over time following administration of control Vehicle, OMTX105 (5 and 20 mg/kg) and OMTX705 (5 and 20 mg/kg).

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Definitions

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Conjugate: As used herein "conjugate" includes the resultant structure formed by linking molecules and specifically includes antibody-drug conjugates (ADCs).

Selectively binds: The terms "selectively binds" and "selective binding" refer to binding of an antibody, or binding fragment thereof, to a predetermined molecule (*e.g.* an antigen) in a specific manner. For example, the antibody, or binding fragment thereof, may bind to FAP, *e.g.* an extracellular portion thereof, with an affinity of at least about 1×10⁷M⁻¹, and may bind to the predetermined molecule with an affinity that is at least two-fold greater (*e.g.* five-fold or ten-fold greater) than its affinity for binding to a molecule other than the predetermined molecule.

### Fibroblast activation protein

As used herein "Fibroblast activation protein", "fibroblast activating protein", "FAP" and "FAPα" are used interchangeably. FAP is also known as prolyl endopeptidase FAP. FAP is a member of the dipeptidyl peptidase (DPP) family, which also comprises DPPIV. FAP is a type II transmembrane glycoprotein comprising a short N terminal cytoplasmic region, a transmembrane region, and a large extracellular region which comprises the active site. FAP monomers are not active, but form active homodimers, as well as heterodimers with DPPIV. FAP expression is typically low in normal adult tissues but is highly upregulated in many cancers, including almost all carcinomas (see *e.g.* Puré and Blomberg, Oncogene (2018) 37(32):4343-4357). FAP expression has also been reported in fibroblast-like synoviocytes (FLSs) in rheumatoid arthritis patients (see, *e.g.,* Bauer et al., Arthritis Res. Therp. (2006):8(6); R171).

The FAP as provided herein includes any forms, homologous or variants of FAP. For example, the FAP may be a FAP of any mammalian species. In some embodiments, FAP is human FAP (also known as Seprase, 170 kDa melanoma membrane-bound gelatinase, fibroblast activation protein alpha or integral membrane serine protease), the amino acid sequence of which is disclosed at UniProt accession No. Q12884 (Version 208, dated 24 January 2024) (SEQ ID NO: 13). In some embodiments, a molecule that binds FAP (*e.g.* an antibody molecule or a conjugate thereof) may bind to a region of the extracellular domain of FAP. The extracellular domain of human FAP comprises residues 26-760 of the full-length human FAP protein (SEQ ID NO:16). In some embodiments, FAP is murine FAP (also known as fibroblast activation protein alpha or integral membrane serine protease), the amino acid sequence of which is disclosed at UniProt accession No. P97321 (Version 180, dated 24 January 2024) (SEQ ID NO:14). The extracellular domain of murine FAP comprises residues 26-761 of the full-length murine FAP protein (SEQ ID NO:16). The FAP according to the present invention is understood to encompass monomeric, dimeric or multimeric forms of FAP.

### Antibody or antigen-binding fragments thereof

The present invention relates to FAP-targeted antibody-drug conjugates comprising anti-FAP antibodies or antigen-binding fragments thereof.

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb), polyclonal antibodies (including polyclonal antisera), monospecific antibodies and multispecific antibodies (*e.g.* bispecific antibodies) and antibody fragments (as long as they display binding to the relevant molecule). Antibodies may be human antibodies, humanised antibodies, or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

Antibodies may be intact, or fragments derived from full antibodies. It has been shown that fragments of a whole antibody can perform the function of binding antigens. Thus, reference to antibody herein, and with reference to the methods, arrays and kits of the invention, covers a full antibody and also covers any polypeptide or protein comprising an antibody binding fragment. Examples of binding fragments are (i) an Fab fragment consisting of VL, VH, CL and CH1 domains; (ii) an Fd fragment consisting of the VH and CH1 domains; (iii) a fragment consisting of the VL and CL domains (the light chain) (iv) a Fv fragment consisting of the VL and VH domains of a single antibody; (v) a dAb fragment which consists of a VH domain; (vi) isolated CDR regions; (vii) F(ab')2 fragments, consisting of a bivalent fragment comprising two linked Fab fragments; (viii) single chain Fv molecules (scFv), wherein a VH domain and a VL domain are linked by a peptide linker which allows the two domains to associate to form an antigen binding site; (ix) bispecific single chain Fv dimers (WO 93/11161) and (x) "diabodies" which consist of multivalent or multispecific fragments constructed by gene fusion (WO94/13804; 58). Fv, scFv or diabody molecules may be stabilised by the incorporation of disulphide bridges linking the VH and VL domains. The term "antibody" as used herein also encompasses minibodies, which comprise a scFv joined to a CH3 domain, and nanobodies, which are antibody fragments derived from heavy-chain only IgG antibodies found in the *Camelidae* family.

Antibodies generally comprise six complementarity-determining regions CDRs; three in the heavy chain variable (VH) region: HC-CDR1, HC-CDR2 and HC-CDR3, and three in the light chain variable (VL) region: LC-CDR1, LC-CDR2, and LC-CDR3. The six CDRs together define the paratope of the antibody, which is the part of the antibody that binds to the target antigen.

The VH region and VL region comprise framework regions (FRs) either side of each CDR, which provide a scaffold for the CDRs. From N-terminus to C-terminus, VH regions comprise the following structure: N term-[HC-FR1]-[HC-CDR1]-[HC-FR2]-[HC-CDR2]-[HC-FR3]-[HC-CDR3]-[HC-FR4]-C term; and VL regions comprise the following structure: N term-[LC-FR1]-[LC-CDR1]-[LC-FR2]-[LC-CDR2]-[LC-FR3]-[LC-CDR3]-[LC-FR4]-C term.

The target antigen for an antibody, or antigen-binding fragment thereof, according to the present invention is fibroblast activation protein (FAP) as described herein.

In some embodiments, an antibody described herein comprises, or consists of, a whole antibody which binds to FAP. As used herein, 'whole antibody' refers to an antibody having a structure which is substantially similar to the structure of an immunoglobulin (Ig). Different kinds of immunoglobulins and their structures are described *e.g.* in Schroeder and Cavacini J Allergy Clin Immunol. (2010) 125(202): S41-S52.

Immunoglobulins of type G *(i.e.* IgG) are -150 kDa glycoproteins comprising two heavy chains and two light chains. From N- to C-terminus, the heavy chains comprise a VH followed by a heavy chain constant region comprising three constant domains (CH1, CH2, and CH3), and similarly the light chains comprise a VL followed by a CL. Depending on the heavy chain, immunoglobulins may be classed as IgG *(e.g.* IgG1, IgG2, IgG3, IgG4), IgA *(e.g.* IgA1, IgA2), IgD, IgE, or IgM. The light chain may be kappa (κ) or lambda (λ).

In some embodiments, the antibody comprises, or consists of, an IgG *(e.g.* IgG1, IgG2, IgG3, IgG4), IgA (e.g. IgA1, IgA2), IgD, IgE, or IgM which binds to FAP.

In some embodiments described herein, an amino acid sequence of an antibody or antigen-binding fragment referred to herein (*e.g.* an amino acid sequence of a CDR or VH/VL region) may comprise one or more amino acid variations. That is, an amino acid sequence of an antibody or antigen-binding fragment thereof referred to herein may comprise one or more variant amino acid residues compared to the amino acid residue at the corresponding position of the equivalent, non-variant amino acid sequence. An amino acid variation as described herein may comprise an amino acid deletion, insertion or substitution. That is a variant amino acid sequence having one amino acid variation, as described herein, may comprise one amino acid deletion, insertion, or substitution as compared to the non-variant reference amino acid sequence.

In some embodiments, a variant as described herein comprises a substitution. For example, in some embodiments, an amino acid sequence of an antibody or antigen-binding fragment referred to herein (e.g. an amino acid sequence of a CDR or VH/VL) may comprise one or more amino acid substitutions. That is, one or more amino acids of an amino acid sequence referred to herein may be substituted with another amino acid. A substitution comprises substitution of an amino acid residue with a non-identical 'replacement' amino acid residue. A replacement amino acid residue of a substitution according to the present disclosure may be a naturally-occurring amino acid residue *(i.e.* encoded by the genetic code) which is non-identical to the amino acid residue at the relevant position of the equivalent, unsubstituted amino acid sequence. In some embodiments, a replacement amino acid may be a non-naturally occurring amino acid residue. Examples of non-naturally occurring amino acid residues include norleucine, ornithine, norvaline, homoserine, aib, and other amino acid residue analogues such as those described in Ellman, et al., Meth. Enzym. 202 (1991) 301-336.

In some embodiments, a substitution may be biochemically conservative. In some embodiments, where an amino acid to be substituted is provided in one of rows 1 to 5 of the table below, the replacement amino acid of the substitution is another, non-identical amino acid provided in the same row:

| **Row** | **Shared property** | **Amino acids** |
|---|---|---|
| 1 | Hydrophobic | Met, Ala, Val, Leu, Ile, Trp, Tyr, Phe, Norleucine |
| 2 | Neutral hydrophilic | Cys, Ser, Thr, Asn, Gln |
| 3 | Acidic or negatively-charged | Asp, Glu |
| 4 | Basic or positively-charged | His, Lys, Arg |
| 5 | Orientation influencing | Gly, Pro |

In some embodiments, a replacement amino acid in a substitution may have the same side chain polarity as the amino acid residue it replaces. In some embodiments, a replacement amino acid in a substitution may have the same side chain charge (at pH 7.4) as the amino acid residue it replaces (see e.g. Alberts et al. Molecular Biology of the Cell. 4th edition. New York: Garland Science; 2002).

In some embodiments, substitution(s) may be functionally conservative. That is, in some embodiments, the substitution may not affect (or may not substantially affect) one or more functional properties (e.g. target binding) of the antigen-binding molecule comprising the substitution as compared to the equivalent unsubstituted molecule.

An antibody or antigen-binding fragment according to the present invention binds FAP. The ability of an antibody or antigen-binding fragment thereof to bind to a given peptide/polypeptide can be analysed by methods well known to the skilled person, including analysis by ELISA, immunoblot (*e.g.* western blot), immunoprecipitation, Surface Plasmon Resonance (SPR; see *e.g.* Hearty et al., Methods Mol Biol (2012) 907:411-442) or Bio-Layer Interferometry (see *e.g.* Lad et al., (2015) J Biomol Screen 20(4): 498-507).

An antibody according to the present invention may bind to human FAP *(i.e.* SEQ ID NO:13). An antibody according to the present invention may bind to murine FAP *(i.e.* SEQ ID NO:14). In some embodiments, an antibody according to the present disclosure displays cross-species reactivity. That is, an antibody according to the present disclosure may bind to FAP from multiple different species. In some embodiments, an antibody according to the present invention binds to human FAP *(i.e.* SEQ ID NO:13) and to murine FAP *(i.e.* SEQ ID NO:14).

In some embodiments, an antibody according to the present invention binds the extracellular region of FAP. For example, an antibody according to the present invention may bind to human FAP in the region corresponding to positions 26 to 760 of SEQ ID NO:13 *(i.e.* SEQ ID NO:15); for example, an antibody according to the present invention may bind to murine FAP in the region corresponding to positions 26 to 761 of SEQ ID NO:14 *(i.e.* SEQ ID NO:16).

An antibody according to the present invention may bind to the extracellular region of human FAP *(i.e.* SEQ ID NO:15) and to the extracellular region of murine FAP *(i.e.* SEQ ID NO:16).

The region of a peptide/polypeptide to which an antibody binds can be determined by the skilled person using various methods well known in the art, including X-ray co-crystallography analysis of antibody-antigen complexes, peptide scanning, mutagenesis mapping, hydrogen-deuterium exchange analysis by mass spectrometry, phage display, competition ELISA and proteolysis-based 'protection' methods. Such methods are described, for example, in Gershoni et al., BioDrugs, 2007, 21(3):145-156.

In some embodiments, an antibody or antigen-binding fragment thereof according to the present invention comprises:
a VH region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9,
or a variant thereof comprising one or two or three amino acid variations in one or more of HC-CDR1, HC-CDR2, or HC-CDR3; and
a VL region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12;
or a variant thereof comprising one or two or three amino acid variations in one or more of LC-CDR1, LC-CDR2 or LC-CDR3.

In some embodiments, an antibody or antigen-binding fragment thereof according to the present invention comprises:
a VH region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9,
or a variant thereof comprising one amino acid variation in one or more of HC-CDR1, HC-CDR2,
or HC-CDR3; and
a VL region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12;
or a variant thereof comprising one amino acid variation in one or more of LC-CDR1, LC-CDR2 or LC-CDR3.

In some embodiments, an antibody or antigen-binding fragment thereof according to the present invention comprises:
a VH region incorporating the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9; and
a VL region incorporating the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12.

In some embodiments, the antibody or antigen-binding fragment thereof comprises:
a VH region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:5.

In some embodiments, the antibody or antigen-binding fragment thereof comprises:
a VL region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:6.

In some embodiments, the antibody or antigen-binding fragment thereof comprises:
a VH region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:5 and a VL region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:6.

In some embodiments, the antibody or antigen-binding fragment thereof comprises a VH region comprising an amino acid sequence having at least 90%, 95% or 99% sequence identity to the amino acid sequence of SEQ ID NO:5.

In some embodiments, the antibody or antigen-binding fragment thereof comprises a VL region comprising an amino acid sequence having at least 90%, 95% or 99% sequence identity to the amino acid sequence of SEQ ID NO:6.

In some embodiments, the antibody or antigen-binding fragment thereof comprises a VH region comprising an amino acid sequence having at least 90%, 95% or 99% sequence identity to the amino acid sequence of SEQ ID NO:5 and a VL region comprising an amino acid sequence having at least 90%, 95% or 99% sequence identity to the amino acid sequence of SEQ ID NO:6.

In some embodiments, the antibody or antigen-binding fragment thereof comprises a VH region comprising the amino acid sequence of SEQ ID NO:5.

In some embodiments, the antibody or antigen-binding fragment thereof comprises a VL region comprising the amino acid sequence of SEQ ID NO:6.

In some embodiments, the antibody or antigen-binding fragment thereof comprises a VH region comprising the amino acid sequence of SEQ ID NO:5 and a VL region comprising the amino acid sequence of SEQ ID NO:6.

In some embodiments, the antibody or antigen-binding fragment thereof comprises a heavy chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:3.

In some embodiments, the antibody or antigen-binding fragment thereof comprises a light chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:4.

In some embodiments, the antibody or antigen-binding fragment thereof comprises a heavy chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:3 and a light chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:4.

In some embodiments, the antibody or antigen-binding fragment thereof comprises a heavy chain comprising an amino acid sequence having at least 90%, 95% or 99% sequence identity with SEQ ID NO:3.

In some embodiments, the antibody or antigen-binding fragment thereof comprises a light chain comprising an amino acid sequence having at least 90%, 95% or 99% sequence identity with SEQ ID NO:4.

In some embodiments, the antibody or antigen-binding fragment thereof comprises a heavy chain comprising an amino acid sequence having at least 90%, 95% or 99% sequence identity with SEQ ID NO:3 and a light chain comprising an amino acid sequence having at least 90%, 95% or 99% sequence identity with SEQ ID NO:4.

In some embodiments, the antibody or antigen-binding fragment thereof comprises a heavy chain comprising the amino acid sequence of SEQ ID NO:3.

In some embodiments, the antibody or antigen-binding fragment thereof comprises a light chain comprising the amino acid sequence of SEQ ID NO:4.

In some embodiments, the antibody or antigen-binding fragment thereof comprises a heavy chain comprising the amino acid sequence of SEQ ID NO:3 and a light chain comprising the amino acid sequence of SEQ ID NO:4.

In some embodiments, the antibody or antigen-binding fragment thereof comprises, or consists of:
one or more (e.g. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:3.

In some embodiments, the antibody or antigen-binding fragment thereof comprises, or consists of:
one or more (e.g. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:4.

In some embodiments, the antibody or antigen-binding fragment thereof comprises, or consists of:
(i) one or more (e.g. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:3; and
(ii) one or more (e.g. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:4.

In some embodiments, the antibody or antigen-binding fragment thereof comprises, or consists of one or more (e.g. two) polypeptides comprising, or consisting of, an amino acid sequence having the amino acid sequence of SEQ ID NO:3.

In some embodiments, the antibody or antigen-binding fragment thereof comprises, or consists of one or more (e.g. two) polypeptides comprising, or consisting of, an amino acid sequence having the amino acid sequence of SEQ ID NO:4.

In some embodiments, the antibody or antigen-binding fragment thereof comprises, or consists of:
(i) one or more (e.g. two) polypeptides comprising, or consisting of, an amino acid sequence having the amino acid sequence of SEQ ID NO:3; and
(ii) one or more (e.g. two) polypeptides comprising, or consisting of, an amino acid sequence having the amino acid sequence of SEQ ID NO:4.

The antibody or antigen-binding fragment thereof according to the invention may be characterised by reference to certain functional properties.

In some embodiments, the antibody or antigen-binding fragment thereof according to the present invention exhibits selective binding to human and mouse FAP. For example, the antibody or antigen-binding fragment thereof according to the present invention demonstrates binding to human and mouse FAP with EC50 values of < 5 nM, < 4 nM, < 3 nM, < 2 nM or < 1 nM. In some embodiments, the antibody or antigen-binding fragment thereof according to the present invention exhibits binding to human and mouse FAP with EC50 values of < 1 nM, < 0.5 nM, < 0.4 nM, or < 0.3 nM.

The binding of an antibody for its target antigen can be determined by the skilled person using various methods well known in the art, including ELISAs, for example as described in Example 2 of the present application.

In some embodiments, the antibody or antigen-binding fragment thereof according to the present invention is internalised into a target cell expressing FAP, following binding of the antibody or antigen-binding fragment thereof to FAP on the target cell's surface. In some embodiments, internalisation of the antibody or antigen-binding fragment thereof according to the present invention, when measured in a suitable internalisation assay, exhibits internalisation into ≥ 50%, ≥60%, ≥ 70%, ≥ 80%, ≥ 85% of target cells within ≤ 120 minutes, ≤ 90 minutes, ≤ 60 minutes of administration of the antibody or antigen-binding fragment thereof. In some embodiments, internalisation of the antibody or antigen-binding fragment thereof into ≥ 85% of target cells occurs in ≤ 60 minutes of administration of the antibody or antigen-binding fragment thereof.

The internalization of an antibody or antigen-binding fragment thereof into target cells can be determined by the skilled person using various methods well known in the art, for example, indirect immunofluorescence (see, *e.g.* Riedl et al. J Biomol Screen. (2016) 21(1):12-23).

The antibody or antigen-binding fragment thereof according to the present invention and their constituent polypeptides may additionally comprise a signal sequence (also known as a leader sequence or signal peptide). Signal sequences normally consist of a sequence of 5-30 hydrophobic amino acids, which form a single alpha helix. Secreted proteins and proteins expressed at the cell surface often comprise signal sequence. Signal sequences are known for many proteins, and are recorded in databases such as GenBank, UniProt and Ensembl, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

The signal sequence may be present at the N-terminus of the polypeptide and may be present in the newly synthesised polypeptide. The signal sequence provides for efficient trafficking of the polypeptide. Signal sequences are often removed by cleavage, and thus are not comprised in the mature polypeptide.

Signal sequences are known for many proteins, and are recorded in databases such as GenBank, UniProt, Swiss-Prot, TrEMBL, Protein Information Resource, Protein Data Bank, Ensembl, and InterPro, and/or can be identified/predicted *e.g.* using amino acid sequence analysis tools such as SignalP (Petersen et al., 2011 Nature Methods 8: 785-786) or Signal-BLAST (Frank and Sippl, 2008 Bioinformatics 24: 2172-2176).

The antibody or antigen-binding fragment thereof according to the present disclosure may comprise the signal sequence shown in SEQ ID NO:17.

### Dolastatins

The present invention relates to a FAP-targeted antibody-drug conjugate comprising a dolastatin.

As used herein, the term "dolastatin" refers to the group of compounds including the natural marine cytotoxic compounds which were first isolated from the sea hare *Dolabella Auricularia,* and their derivatives.

Dolastatins include dolastatin 10 and dolastatin 15. These structurally similar dolastatins inhibit cell proliferation and induce apoptosis. Dolastatin 10 is more potent than dolastatin 15, although both compounds demonstrate cytotoxicity at nanomolar concentrations (see, for example, Singh et al. J Nat Prod. (2022) 85(3):666-687 and Bai et al. Biochem Pharmacol. (1992) 43(12):2637-45). In some embodiments, the dolastatin according to the present invention is dolastatin 10 or a derivative thereof. In some embodiments, the dolastatin is dolastatin 15 or a derivative thereof.

Dolastatin 10 inhibits tubulin polymerisation and tubulin-dependent guanosine triphosphate hydrolysis, thereby disrupting microtubule dynamics and blocking mitosis. Dolastatin 15 shows significantly weaker binding to tubulin than dolastatin 10, indicating an alternative mechanism for dolastatin 15's potent cytotoxicity.

In some embodiments, the dolastatin according to the present invention is a dolastatin 15 derivative. Dolastatin 15 derivatives include, for example, ILX651 (also known as tasidotin), LU103793 (also known as Cematodin^{®}).

In some embodiments, the dolastatin according to the present invention is a dolastatin 10 derivative. Dolastatin 10 derivatives include, for example, monomethyl auristatin E (MMAE), monomethyl auristatin F (MMAF), monomethyl auristatin D (MMAD), Auristatin PE (TZT-1027, also known as Soblidotin, Drugbank accession number # DB12677), Auristatin PYE, mono methyl autistatin PYE (MMAPYE). In some embodiments, the dolastatin is MMAE or MMAF. In preferred embodiments, the dolastatin according to the present invention is MMAE.

In some embodiments, the dolastatin according to the present invention is MMAE or a pharmaceutically acceptable salt, hydrate, solvate, or crystalline form thereof. In some embodiments, the dolastatin has the following structure:

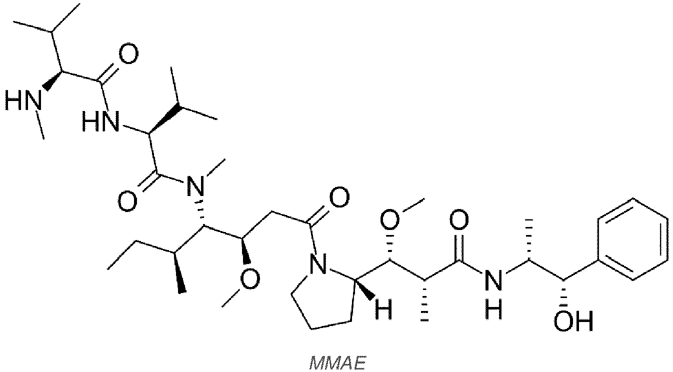

### Linker

The term "linker" as used herein refers to the component linking the antibody and the drug. That is, the linker according to the present invention refers to the component of the conjugate of the present invention that connects the anti-FAP antibody and the dolastatin.

In some embodiments, the linker according to the present invention is a cleavable linker. Cleavable linkers are cleaved to release the drug payload *(i.e.* the dolastatin) to the target location *(i.e.* the target cell, tissue *etc*.).

In some embodiments, the linker according to the present invention is an enzymatically cleavable linker. In some embodiments, the linker is a peptide-based linker. In some embodiments, the linker is a protease-cleavable linker, optionally a lysosomal protease-cleavable linker. In some embodiments, the linker is cleaved by an intracellular protease. In some embodiments, the linker is cleaved by a cathepsin.

In some embodiments, the linker according to the present invention comprises a valine-citrulline unit. Valine-citrulline (Val-Cit) is a protease sensitive dipeptide that is cleaved intracellularly by cathepsins *(i.e.* cathepsin B).

In some embodiments, the linker according to the present invention comprises a self-immolative group. That is, in some embodiments, the linker comprises a group that undergoes self-immolative elimination to facilitate release of the drug payload *(i.e.* the dolastatin) to the target location *(i.e.* the target cell, tissue *etc*.). In some embodiments, the linker comprises a para-aminobenzylcarbamate unit.

In some embodiments, the linker is coupled to the antibody via cysteine-based conjugation. In some embodiments, the linker comprises a thiol-reactive group. In some embodiments, the linker is coupled to the antibody via reaction between cysteine residues in the antibody and a thiol-reactive functional group in the linker. In some embodiments the linker comprises a maleimide group. In some embodiments, the linker comprises a maleimidocaproyl group. The maleimidocaproyl group according to the present invention is reacted with a thiol group of a cysteine residue of the antibody according to the present invention to form a sulphur-carbon bond, thereby effecting linkage of the linker to the antibody.

In some embodiments, the linker according to the present invention further comprises a spacer. Spacers are sometimes required due to the bulky nature of payload moieties. Commonly employed spacer moieties include hemiaminal groups, PEG groups, polar acyl sulfamide groups, polar carbamoyl sulfamide groups and HydraSpace (described *e.g.* in Verkade et al., Antibodies (Basel) (2018) 7(1):12 and WO 2016/053107 A1).

In some embodiments, the spacer has a chain length of 2 to 30 atoms. In some embodiments, the spacer comprises or consists of an alkylene *(i.e.* divalent alkyl) or heteroalkylene *(i.e.* divalent heteroalkyl) group. In some embodiments, the spacer comprises or consists of an alkylene or oxyalkylene group. In some embodiments, the spacer comprises or consists of a group (CH₂)ₙ or (OCH₂CH₂)ₙ, wherein n ≥ 1. In some embodiments, the spacer comprises or consists of a group (OCH₂CH₂)ₙ, wherein n ≥ 1. In particular, n may be 1 to 15, 1 to 10, 1 to 6, or 2 to 5. For example, n may be 3 or 4. In some embodiments, the spacer comprises between one and six ethylene glycol units, *e.g.* a triethylene glycol.

### Anti-cancer agents

The term "anti-cancer agents" as provided herein refers to biologically active agents which are useful in the treatment of cancer.

In some embodiments, in accordance with any aspect of the present invention, the conjugate of the invention may be administered with, or may be for administration with, (whether simultaneously, sequentially or separately) other anti-cancer drugs, including, but not limited to, a cytotoxic chemotherapeutic agent or an anti-angiogenic agent or an immunotherapeutic agent.

Cytotoxic chemotherapeutic agents are well known in the art and include agents such as:
Alkylating agents including nitrogen mustards (for example, bendamustine, chlorambucil, cyclophosphamide, ifosfamide, mechlorethamine/chlormethine, melphalan, uramustine); ethylenimines and methylmelamine derivatives (for example altretamine, thiotepa); expoxides (for example dianhydrogalacititol, dibromodulcitol), alkyl sulfonates (for example, busulfan, hepsulfan), nitrosoureas (for example, carmustine, lomustine, streptozocin, semustine), triazenes (for example dacarbazine, procarbazine, temozolomide), quinone-containing/bioreductive alkylating agents (for example, mitomycin C). Platinum-based chemotherapeutic agents or platinum-based anti-neoplastic agents (also known as "alkylating-like" agents) including Cisplatin, Carboplatin, Dicycloplatin, Eptaplatin, Lobaplatin, Miriplatin, Nedaplatin, Oxaliplatin, Picoplatin, Satraplatin, Triplatin tetranitrate. Antimetabolites including folic acid analogues such as methotrexate (amethopterin); pyrimidine analogues such as fluorouracil (5-fluorouracil; 5-FU), floxuridine (fluorodeoxyuridine; FudR) and cytarabine (cytosine arabinoside); and purine analogues and related inhibitors such as mercaptopurine (6-mercaptopurine; 6-MP), thioguanine (6-thioguanine; TG) and pentostatin (2'-deoxycofonnycin). Natural Products including vinca alkaloids such as vinblastine (VLB), vincristine and vinorelbine; antibiotics such as anthracenediones (e.g. mitoxantrone and anthracycline), dactinomycin (actinomycin D), daunorabicin (daunomycin; rubidomycin), doxorubicin, bleomycin, plicamycin (mithramycin) and mitomycin (mitomycin Q; enzymes such as L-asparaginase; and biological response modifiers such as interferon alphenomes. Monoclonal antibodies including Alemtuzumab, Atezolizumab, Avelumab, Belantamab, Bevacizumab, Blinatumomab, Brentuximab, Cemiplimab, Cetuximab, Daratumumab, Dinutuximab, Dostarlimab, Durvalumab, Elotuzumab, Enfortumab, Gemtuzumab, Inotuzumab Ozogamicin, Ipilimumab, Mogamulizumab, Moxetumomab Pasudotox, Necitumumab, Nivolumab, Ofatumumab, Olaratumab, Panitumumab, Pembrolizumab, Pertuzumab, Polatuzumab Vedotin, Ramucirumab, Rituximab, Sacituzumab Govitecan, Teclistamab, Tisotumab Vedotin, Tositumomab, Trastuzumab, Trastuzumab Deruxtecam, Trastuzumab Emtansine, Tremelimumab. Protein kinase inhibitors including Abemaciclib, Acalabrutinib, Adagrasib, Afatinib, Alectinib, Alpelisib, Asciminib, Axitinib, Binimetinib, Bortezomib, Bosutinib, Brigatinib, Cabozantinib, Carfilzomib, Ceritinib, Cobimetinib, Copanlisib, Crizotinib, Dabrafenib, Dacomitinib, Dasatinib, Duvelisib, Enasidenib, Encorafenib, Entrectinib, Erdafitinib, Erlotinib, Fedratinib, Futibatinib, Gefitinib, Gilteritinib, Glasdegib, Ibrutinib, Idelalisib, Imatinib, Infigratinib, Ivosidenib, Ixazomib, Lapatinib, Larotrectinib, Lenvatinib, Lorlatinib, Midostaurin, Mobocertinib, Neratinib, Nilotinib, Niraparib, Olaparib, Olutasidenib, Osimertinib, Palbociclib, Pazopanib, Pemigatinib, Pexidartinib, Ponatinib, Regorafenib, Ribocicib, Ripretinib, Rucaparib, Ruxolitinib, Selumetinib, Sonidegib, Sorafenib, Sunitinib, Talazoparib, Trametinib, Trilaciblib, Umbralisib, Vandetanib, Vemurafenib, Vismodegib, Zanubrutinib. Taxanes including paclitaxel (also known as Taxol) and docetaxel (also known as Taxotere), cabazitaxel (also known as Jevtana). Topoisomerase inhibitors including etoposide, irinotecan, teniposide, topotecan. Hormone agonists/antagonists including antiandrogens (*e.g.* Abiraterone, Apalutamide, Bicalutamide, Cyproterone, Enzalutamide, Flutamide, Nilutamide flutamide and tamoxifen); antiestrogens (*e.g.* aromatase inhibitors, Anastrozole, Exemestane, Fulvestrant, Letrozole, Raloxifene, Tamoxifen, Toremifene); gonadotropin releasing hormone analogues (*e.g.* Degarelix, Goserelin, Histrelin, Leuprolide, Relugolix, Triptorelin); and peptide hormones (*e.g.* Lanreotide, Octreotide, Pasireotide). Miscellaneous agents including substituted urea such as hydroxyurea; and adrenocortical suppressant such as mitotane (o, p'-DDD) and aminoglutethimide.

Anti-angiogenic agents are well known in the art and include anti-cancer agents such as bevacizumab, itraconazole, and carboxyamidotriazole.

Immunotherapeutic agents are known in the art and include, for example, anti-programmed cell death protein 1 (PD-1) antibodies and anti-programmed death-ligand 1 (PD-L1) antibodies, including Nivolumab (MDX1106) and Pembrolizumab (MK-3475).

### Subject

The mammalian subject in accordance with aspects of the present disclosure may be any mammal or human. A subject may therefore be a rat, mouse, feline, canine, equine, porcine, ovine, bovine, primate or human. The subject is preferably human. The subject may be male or female. The subject may be a patient. A subject may have been diagnosed with a disease/condition described herein requiring treatment, may be suspected of having such a disease/condition, or may be at risk of developing/contracting such a disease/condition.

In some embodiments, the subject may be a human diagnosed with or classified as being at risk of developing a cancer. In some embodiments, the subject is a human patient having cancer. In certain embodiments, the subject may be a laboratory animal, *e.g.,* a mouse model of a cancer.

A cancer may be any cancer. In some embodiments a cancer may comprise a solid tumor, for example, a colorectal tumor, a fibrosarcoma, a gastric tumor, a glioblastoma, a renal tumor, a hepatic tumor, a pulmonary tumor, a melanoma, a nasopharyngeal tumor, an oral tumor, an osteosarcoma, an ovarian tumor, a pancreatic tumor, a brain tumor, or a prostatic tumor. Alternatively, the cancer may comprise a blood cancer or haematological cancer, such as a lymphoma, a leukaemia, or a myeloma.

In particular, the cancer may be a pancreatic cancer, sarcoma (for example, fibrosarcoma), oesophageal cancer, breast cancer, melanoma, lung cancer, gastric cancer, head and neck cancer, ovarian cancer, bladder cancer or colorectal cancer. The cancer may be exocrine pancreatic cancer. In particular, the cancer may be pancreatic ductal adenocarcinoma.

In some embodiments, the cancer is a cancer expressing FAP. In some embodiments, the cancer is a cancer with upregulated FAP expression (*i.e.* in comparison with a comparable non-cancerous cell/tissue/organ/organ system). In some embodiments, a "cancer expressing FAP" includes any cancer comprising cells/tissue/stroma/tumor microenvironment encoding or expressing FAP. In some embodiments, a "cancer with upregulated FAP expression" includes any cancer comprising cells/tissue/stroma/tumor microenvironment expressing FAP at an upregulated level, e.g. as compared to the level of expression by comparable non-cancerous cells/tissue/stroma/tumor microenvironment.

In certain embodiments, the subject may be a mammal (*e.g.* a human) that has been diagnosed with or classified as being at risk of developing an inflammatory condition, such as rheumatoid arthritis (RA). In particular, the subject may be a human having RA.

### Treatment

Conjugates according to the present invention and compositions comprising such agents may be provided for use in methods of medical treatment. Treatment may be provided to subjects having a disease or condition in need of treatment. The disease or condition may be a cancer.

In some embodiments, the cancer may comprise cells/tissue/stroma/tumor microenvironment which expresses FAP. For example, the cancer may be treated by targeted delivery of the cytotoxic payload of the present invention *(i.e.* the dolastatin) by specific recognition of FAP within cells/tissue/stroma/tumor microenvironment of the cancer by the anti-FAP antibody of the invention. In some embodiments, the antibody or antigen-binding fragment thereof according to the present invention binding to FAP may facilitate receptor-mediated internalisation of the conjugate of the present invention, which may then be cleaved by intracellular proteases to release dolastatin, thereby inducing cytotoxicity.

The treatment may be aimed at prevention of the development or progression of a cancer. As such, the conjugates may be used to formulate pharmaceutical compositions or medicaments and subjects may be prophylactically treated against development of a disease state. This may take place before the onset of symptoms of the disease state, and/or may be given to subjects considered to be at greater risk of development or progression of cancer.

It will be clear to the person skilled in the art that the therapeutic utility of the present invention extends to essentially any disease/condition which would benefit from targeted killing of FAP-expressing cells. The therapeutic utility of the present invention extends to any subject suffering from a condition comprising cells with upregulated FAP expression *(i.e.* as compared to equivalent cells in a healthy subject).

A cancer may be determined to express FAP by any suitable means, which are well known to the skilled person, e.g. based on analysis of a biological sample. A cancer expressing FAP may be identified by detection of expression of FAP. Expression may be gene expression or protein expression. Gene expression can be determined *e.g.* by detection of mRNA encoding FAP, for example by quantitative real-time PCR (qRT-PCR). Protein expression can be determined *e.g.* by detection of FAP, for example by antibody-based methods, for example by western blot, immunohistochemistry, immunocytochemistry, flow cytometry, ELISA. Herein, "a cancer expressing FAP" includes any cancer comprising cells/tissue/stroma/tumor microenvironment encoding or expressing FAP. In some embodiments, the cells/tissue/stroma may be a cells/tissue/stroma of a tumor.

In some embodiments, the anti-FAP conjugates described herein may be for useful in the treatment of an inflammatory disease. FAP has been associated with fibrosis, in particular fibrotic conditions involved the liver *(e.g.* cirrhosis), lung *(e.g.* idiopathic pulmonary fibrosis) and colon *(e.g.* Crohn's disease). FAP has also been associated with arthritis, in particular rheumatoid arthritis.

In some embodiments, the conjugate according to the present invention is for use in a method of treating an inflammatory disease. In some embodiments, the inflammatory disease is fibrosis. The fibrosis may be of the liver, lung and/or colon. In some embodiments, the inflammatory disease is arthritis, in particular rheumatoid arthritis.

### Dosages and method of administration

Multiple doses of the conjugate may be provided. One or more, or each, of the doses may be accompanied by simultaneous, sequential or separate administration of another anti-cancer agent.

In some embodiments the time interval between administration steps performed sequentially is one of at least 12 hours, 24 hours, 36 hours, 48 hours, 72 hours, 4 days, 5 days, 7 days, 10 days, 14 days, 18 days, 21 days, or 28 days, or 1, 2, 3, 4, 5, or 6 months.

In embodiments where administration steps comprise plural, separate introductions of material into a subject, time intervals provided herein may be between the final introduction of one administration step, and the first introduction of the subsequent administration step.

In some embodiments, administration steps of the methods of the present disclosure may be performed simultaneously. That is, in some embodiments an administration step as described herein is performed at the same time as or immediately before/after another administration step as described herein. The time interval between administration steps performed simultaneously is preferably less than one of 72 hours, 48 hours, 36 hours, 24 hours, 12 hours, or 6 hours.

Preferably, the conjugate of the present invention, or a pharmaceutical composition comprising the conjugate according to the present invention is administered via injection. Such administration may be both via infusion (continuous) or bolus (discrete) administration. The method of administration via injection may be, for example, subcutaneous, intradermal, intramuscular, intravenous, intraarterial, intracardiac, intrathecal, intraspinal, intracapsular, subcapsular, intra-orbital, intraperitoneal, intratracheal, subcuticular, intraarticular, subarachnoid, and intra-sternal injection. Preferably, the administration is by intravenous infusion or intravenous injection (bolus administration). More preferably, the administration is by intravenous infusion.

Administration of the articles according to the present disclosure is preferably in a 'therapeutically-effective' or 'prophylactically-effective' amount, this being sufficient to show therapeutic/prophylactic benefit to the subject. The actual amount administered, and rate and time-course of administration, will depend on the nature and severity of the disease/condition and the particular article administered. Prescription of treatment, *e.g.* decisions on dosage *etc.,* is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

### Pharmaceutical compositions

The conjugates of the present invention may be provided in the form of a composition comprising the conjugate. Such compositions may be pharmaceutical compositions or medicaments suitable for clinical use, and may additionally comprise pharmaceutically-acceptable carriers, diluents, excipients or adjuvants.

A pharmaceutical composition according to the present invention may comprise, in addition to the conjugate as described herein, one or more other pharmaceutically acceptable ingredients well known to those skilled in the art, including, but not limited to: pharmaceutically acceptable carriers, diluents, excipients, adjuvants, buffers, pH modifiers, preservatives, anti-oxidants, bacteriostats, stabilisers, suspending agents, solubilisers, surfactants (*e.g.,* wetting agents), colouring agents, and isotonicising solutes (i.e., which render the formulation isotonic with the blood, or other relevant bodily fluid, of the intended recipient).

The term 'pharmaceutically-acceptable' as used herein pertains to compounds, ingredients, materials, compositions, dosage forms, etc., which are, within the scope of sound medical judgment, suitable for use in contact with the tissues of the subject in question (*e.g.* a human subject) without excessive toxicity, irritation, allergic response, or other problem or complication, commensurate with a reasonable benefit/risk ratio. Each carrier, diluent, excipient, adjuvant, filler, buffer, preservative, anti-oxidant, lubricant, binder, stabiliser, solubiliser, surfactant, masking agent, colouring agent, flavouring agent or sweetening agent of a composition according to the present disclosure must also be 'acceptable' in the sense of being compatible with the other ingredients of the formulation. Suitable carriers, diluents, excipients, adjuvants, fillers, buffers, preservatives, anti-oxidants, lubricants, binders, stabilisers, solubilisers, surfactants, masking agents, colouring agents, flavouring agents or sweetening agents can be found in standard pharmaceutical texts, for example, Remington's 'The Science and Practice of Pharmacy' (Ed. A. Adejare), 23rd Edition (2020), Academic Press.

A pharmaceutically acceptable excipient may be a compound or a combination of compounds entering into a pharmaceutical composition which does not provoke secondary reactions and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

The pharmaceutical compositions/medicaments according to the present disclosure may be formulated for administration to a subject, *e.g.* administration via a route of administration as appropriate for the nature of the therapeutic agent and the disease to be treated/prevented. In some embodiments, a pharmaceutical composition/medicament may be formulated for parenteral, systemic, intravascular, intravenous, intra-arterial, intramuscular, topical, intracavitary, intrathecal, intraocular, intraconjunctival, intratumoral, subcutaneous, intradermal, oral or transdermal administration. In some embodiments, a pharmaceutical composition/medicament may be formulated for administration by injection or infusion, or administration by ingestion.

For intra-venous administration, *e.g.* by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEENTM, PLURONICSTM or polyethylene glycol (PEG).

### Sequences

| **SEQ ID NO** | **DESRIPTION** | **SEQUENCE** |
|---|---|---|
| 1 | Hu36 IgG1-HC with signal sequence | METDTLLLWVLLLWVPGSTGQVQLVQSGAEVKKPGASVKVSCKASGYTF TENIIHWVRQAPGQGLEWMGWFHPGSGSIKYNEKFKDRVTMTADTSTST VYMELSSLRSEDTAVYYCARHGGTGRGAMDYWGQGTLVTVSSASTKGP SVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKT HTCPPCPAPPVAGPSVFLFPPKPKDTLMISRTPEVTCVVVDVSHEDPEVK FNWYVDGVEVHNAKTKPREEQYNSTYRVVSVLTVLHQDWLNGKEYKCK VSNKGLPSSIEKTISKAKGQPREPQVYTLPPSRDELTKNQVSLTCLVKGFY PSDIAVEWESNGQPENNYKTTPPVLDSDGSFFLYSKLTVDKSRWQQGNV FSCSVMHEALHNHYTQKSLSLSPGK |
| 2 | Hu36 IgG1-LC with signal sequence | METDTLLLWVLLLWVPGSTGDIQMTQSPSSLSASVGDRVTITCRASKSVS TSAYSYMHWYQQKPGKAPKLLIYLASNLESGVPSRFSGSGSGTDFTLTIS SLQPEDFATYYCQHSRELPYTFGQGTKLEIKRTVAAPSVFIFPPSDEQLKS GTASVVCLLNNFYPREAKVQWKVDNALQSGNSQESVTEQDSKDSTYSLS STLTLSKADYEKHKVYACEVTHQGLSSPVTKSFNRGEC |
| 3 | Hu36 IgG1-HC | QVQLVQSGAEVKKPGASVKVSCKASGYTFTENIIHWVRQAPGQGLEWM GWFHPGSGSIKYNEKFKDRVTMTADTSTSTVYMELSSLRSEDTAVYYCA RHGGTGRGAMDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPPVAGPSVFLF PPKPKDTLMISRTPEVTCVVVDVSHEDPEVKFNWYVDGVEVHNAKTKPR EEQYNSTYRVVSVLTVLHQDWLNGKEYKCKVSNKGLPSSIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 4 | Hu36 IgG1-LC | DIQMTQSPSSLSASVGDRVTITCRASKSVSTSAYSYMHWYQQKPGKAPK LLIYLASNLESGVPSRFSGSGSGTDFTLTISSLQPEDFATYYCQHSRELPY |
| | | TFGQGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEV THQGLSSPVTKSFNRGEC |
| 5 | Hu36 IgG1 VH | QVQLVQSGAEVKKPGASVKVSCKASGYTFTENIIHWVRQAPGQGLEWM GWFHPGSGSIKYNEKFKDRVTMTADTSTSTVYMELSSLRSEDTAVYYCA RHGGTGRGAMDYWGQGTLVTVSS |
| 6 | Hu36 IgG1 VL | DIQMTQSPSSLSASVGDRVTITCRASKSVSTSAYSYMHWYQQKPGKAPK LLIYLASNLESGVPSRFSGSGSGTDFTLTISSLQPEDFATYYCQHSRELPY TFGQGTKLEIKR |
| 7 | Hu36 IgG1 HC-CDR1 | ENIIH |
| 8 | Hu36 IgG1 HC-CDR2 | WFHPGSGSIKYNEKFKD |
| 9 | Hu36 IgG1 HC-CDR3 | HGGTGRGAMDY |
| 10 | Hu36 IgG1 LC-CDR1 | RASKSVSTSAYSYMH |
| 11 | Hu36 IgG1 LC-CDR2 | LASNLES |
| 12 | Hu36 IgG1 LC-CDR3 | QHSRELPYT |
| 13 | Human FAP (Uniprot #Q12884) | MKTWVKIVFGVATSAVLALLVMCIVLRPSRVHNSEENTMRALTLKDILNGT FSYKTFFPNWISGQEYLHQSADNNIVLYNIETGQSYTILSNRTMKSVNASN YGLSPDRQFVYLESDYSKLWRYSYTATYYIYDLSNGEFVRGNELPRPIQY LCWSPVGSKLAYVYQNNIYLKQRPGDPPFQITFNGRENKIFNGIPDWVYE EEMLATKYALWWSPNGKFLAYAEFNDTDIPVIAYSYYGDEQYPRTINIPYP KAGAKNPVVRIFIIDTTYPAYVGPQEVPVPAMIASSDYYFSWLTWVTDERV CLQWLKRVQNVSVLSICDFREDWQTWDCPKTQEHIEESRTGWAGGFFV STPVFSYDAISYYKIFSDKDGYKHIHYIKDTVENAIQITSGKWEAINIFRVTQ DSLFYSSNEFEEYPGRRNIYRISIGSYPPSKKCVTCHLRKERCQYYTASFS DYAKYYALVCYGPGIPISTLHDGRTDQEIKILEENKELENALKNIQLPKEEIK KLEVDEITLWYKMILPPQFDRSKKYPLLIQVYGGPCSQSVRSVFAVNWISY LASKEGMVIALVDGRGTAFQGDKLLYAVYRKLGVYEVEDQITAVRKFIEM GFIDEKRIAIWGWSYGGYVSSLALASGTGLFKCGIAVAPVSSWEYYASVY TERFMGLPTKDDNLEHYKNSTVMARAEYFRNVDYLLIHGTADDNVHFQN SAQIAKALVNAQVDFQAMWYSDQNHGLSGLSTNHLYTHMTHFLKQCFSL SD |
| 14 | Murine FAP (Uniprot # P97321) | MKTWLKTVFGVTTLAALALVVICIVLRPSRVYKPEGNTKRALTLKDILNGTF SYKTYFPNWISEQEYLHQSEDDNIVFYNIETRESYIILSNSTMKSVNATDYG LSPDRQFVYLESDYSKLWRYSYTATYYIYDLQNGEFVRGYELPRPIQYLC WSPVGSKLAYVYQNNIYLKQRPGDPPFQITYTGRENRIFNGIPDWVYEEE |
| | | MLATKYALWWSPDGKFLAYVEFNDSDIPIIAYSYYGDGQYPRTINIPYPKA GAKNPVVRVFIVDTTYPHHVGPMEVPVPEMIASSDYYFSWLTWVSSERV CLQWLKRVQNVSVLSICDFREDWHAWECPKNQEHVEESRTGWAGGFFV STPAFSQDATSYYKIFSDKDGYKHIHYIKDTVENAIQITSGKWEAIYIFRVTQ DSLFYSSNEFEGYPGRRNIYRISIGNSPPSKKCVTCHLRKERCQYYTASFS YKAKYYALVCYGPGLPISTLHDGRTDQEIQVLEENKELENSLRNIQLPKVEI KKLKDGGLTFWYKMILPPQFDRSKKYPLLIQVYGGPCSQSVKSVFAVNWI TYLASKEGIVIALVDGRGTAFQGDKFLHAVYRKLGVYEVEDQLTAVRKFIE MGFIDEERIAIWGWSYGGYVSSLALASGTGLFKCGIAVAPVSSWEYYASIY SERFMGLPTKDDNLEHYKNSTVMARAEYFRNVDYLLIHGTADDNVHFQN SAQIAKALVNAQVDFQAMWYSDQNHGISSGRSQNHLYTHMTHFLKQCFS LSD |
| 15 | Human FAP extracellular region (Uniprot #Q12884, positions 26 to 760) | LRPSRVHNSEENTMRALTLKDILNGTFSYKTFFPNWISGQEYLHQSADNNI VLYNIETGQSYTILSNRTMKSVNASNYGLSPDRQFVYLESDYSKLWRYSY TATYYIYDLSNGEFVRGNELPRPIQYLCWSPVGSKLAYVYQNNIYLKQRP GDPPFQITFNGRENKIFNGIPDWVYEEEMLATKYALWWSPNGKFLAYAEF NDTDIPVIAYSYYGDEQYPRTINIPYPKAGAKNPVVRIFIIDTTYPAYVGPQE VPVPAMIASSDYYFSWLTWVTDERVCLQWLKRVQNVSVLSICDFREDWQ TWDCPKTQEHIEESRTGWAGGFFVSTPVFSYDAISYYKIFSDKDGYKHIH YIKDTVENAIQITSGKWEAINIFRVTQDSLFYSSNEFEEYPGRRNIYRISIGS YPPSKKCVTCHLRKERCQYYTASFSDYAKYYALVCYGPGIPISTLHDGRT DQEIKILEENKELENALKNIQLPKEEIKKLEVDEITLWYKMILPPQFDRSKKY PLLIQVYGGPCSQSVRSVFAVNWISYLASKEGMVIALVDGRGTAFQGDKL LYAVYRKLGVYEVEDQITAVRKFIEMGFIDEKRIAIWGWSYGGYVSSLALA SGTGLFKCGIAVAPVSSWEYYASVYTERFMGLPTKDDNLEHYKNSTVMA RAEYFRNVDYLLIHGTADDNVHFQNSAQIAKALVNAQVDFQAMWYSDQN HGLSGLSTNHLYTHMTHFLKQCFSLSD |
| 16 | Murine FAP extracellular region (Uniprot #P97321 positions 26 to 761) | LRPSRVYKPEGNTKRALTLKDILNGTFSYKTYFPNWISEQEYLHQSEDDNI VFYNIETRESYIILSNSTMKSVNATDYGLSPDRQFVYLESDYSKLWRYSYT ATYYIYDLQNGEFVRGYELPRPIQYLCWSPVGSKLAYVYQNNIYLKQRPG DPPFQITYTGRENRIFNGIPDWVYEEEMLATKYALWWSPDGKFLAYVEFN DSDIPIIAYSYYGDGQYPRTINIPYPKAGAKNPVVRVFIVDTTYPHHVGPME VPVPEMIASSDYYFSWLTWVSSERVCLQWLKRVQNVSVLSICDFREDWH AWECPKNQEHVEESRTGWAGGFFVSTPAFSQDATSYYKIFSDKDGYKHI HYIKDTVENAIQITSGKWEAIYIFRVTQDSLFYSSNEFEGYPGRRNIYRISIG NSPPSKKCVTCHLRKERCQYYTASFSYKAKYYALVCYGPGLPISTLHDGR TDQEIQVLEENKELENSLRNIQLPKVEIKKLKDGGLTFWYKMILPPQFDRS KKYPLLIQVYGGPCSQSVKSVFAVNWITYLASKEGIVIALVDGRGTAFQGD KFLHAVYRKLGVYEVEDQLTAVRKFIEMGFIDEERIAIWGWSYGGYVSSLA LASGTGLFKCGIAVAPVSSWEYYASIYSERFMGLPTKDDNLEHYKNSTVM ARAEYFRNVDYLLIHGTADDNVHFQNSAQIAKALVNAQVDFQAMWYSDQ NHGISSGRSQNHLYTHMTHFLKQCFSLSD |
| 17 | Signal sequence | METDTLLLWVLLLWVPGSTG |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1: Comparative in vitro activity of MMAE versus cytolysin payload

The cytotoxic activity of A1B1 cytolysin and MMAE payloads was analyzed via cell-viability assay using the colorectal cancer cell lines HCT15 (high Pgp expression) and HCT116 (low Pgp expression). The day before the assay, cells were seeded and cultivated overnight at 37 °C. Titration of the payloads diluted in medium was added to the cells and incubated for 48 hours at 37 °C. Finally, cell viability was analyzed by crystal violet staining.

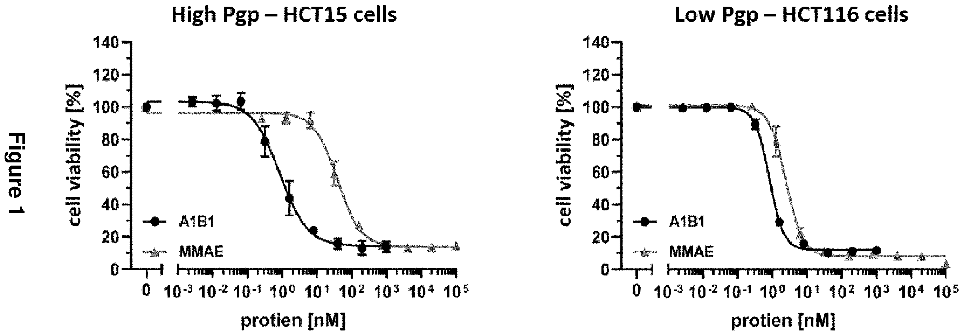

Both payloads showed a concentration-dependent killing of both cell lines (HCT15 and HCT116). A1B1 showed strongest killing on both cell lines with IC50 values of 0.9 nM for HCT15 and 0.8 nM for HCT116. MMAE showed lower activity than A1B1 cytolysin on both cell lines, independently of Pgp levels, with IC50 values of 40 nM (in HCT15) and 3 nM in HCT116. (Figure 1)

**Table 1: Bioactivity of AlBland MMAE on HCT15 and HCT116 cell lines.**

| **Payloads** | **IC 50 HCT15** | **IC50 HCT116** |
|---|---|---|
| A1B1 | 0.9 ± 0.3 nM | 0.8 ± 0.1 nM |
| MMAE | 39.5 ± 10.1 nM | 2.5 ± 0.4 nM |

### EXAMPLE 2: Comparative in vitro properties of MMAE versus cytolysin anti-FAP antibody drug conjugates

Generation and production of humanized anti-FAP antibody was carried out as described in Example 1 of WO 2015/118030 A2.

### Generation and production of anti-FAP MMAE antibody drug conjugate

OMTX705 was produced as described in Example 5 of WO 2015/118030 A2.

For the obtention of OMTX105 antibody-drug conjugate (ADC), the anti-FAP IgG antibody described previously was conjugated with the drug-linker vcMMAE, through a non-site specific and thiol-based reaction on the cysteine residues of the antibody.

The antibody was reduced by 2.10 TCEP/mAb equivalents for 2 hours and conjugated with 8.0 MMAE/mAb equivalents for 1 hour. The conjugation was followed by Ultrafiltration and formulation in 20 mM Histidine/Histidine-HCl, 8% sucrose, pH 6.0. The final protein was concentrated to 12.10 mg/mL in formulation buffer.

The final OMTX105 ADC product obtained presented a drug-antibody ratio (DAR) of 3.92 analyzed by HIC-HPLC. The purity was 99.61% of main peak and 0.39% of HMW. Free drug analyzed by HIC-HPLC was <0.15% and endotoxin content was 0.223 EU/mL

**Table 3: Main characteristics of OMTX105 and OMTX705 Antibody-Drug Conjugates**

| **ADC** | **Conc. (mg/mL)** | **HIC-DAR** | **Monomer %** | **HMW%** | **Free drug (mol/mol %)** | **Endo (EU/mg)** |
|---|---|---|---|---|---|---|
| OMTX705 | 27.1 | 3.8 | 97 | 0.6 | nd. | 0.96 |
| OMTX105 | 12.10 | 3.92 | 99.61 | 0.39 | <0.15 | 0.223 |

| | | | | | | |
|---|---|---|---|---|---|---|
| nd =not detected | | | | | | |

### In vitro binding and activity of anti-FAP MMAE vs cytolysin conjugates

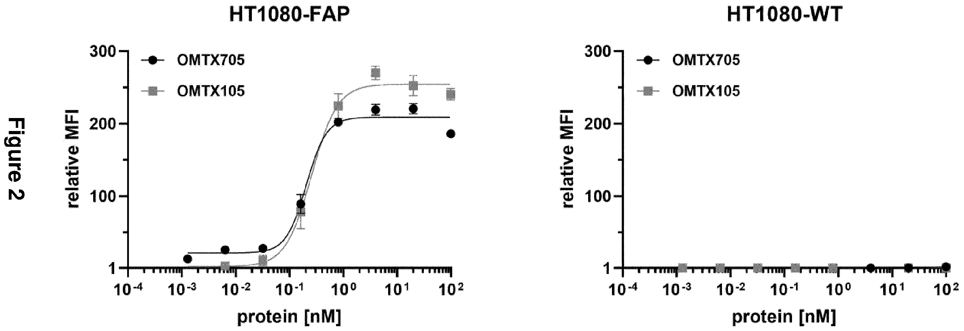

FAP-specific cell binding of OMTX105 compared to OMTX705 was analyzed via flow cytometry using FAP-expressing HT1080-FAP and FAP-negative HT1080-WT cells. Bound antibodies were detected using PE-labeled anti-human Fc secondary antibody and median fluorescence intensity (MFI) was measured. OMTX105 showed a concentration-dependent binding to HT1080-FAP cells with EC50 values of 255.0 pM, similar to OMTX705 with values of 203.9pM (Figure 2). No binding was detected in HT1080-WT for either of the two ADCs.

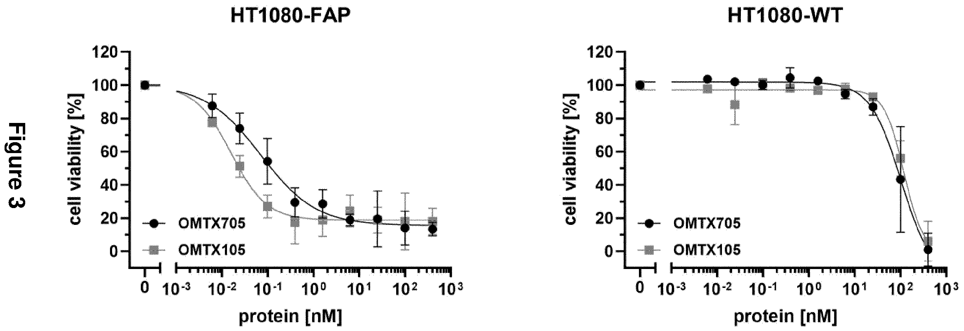

OMTX105 cytotoxic activity was analyzed via cell viability assay using HT1080-FAP and HT1080-WT cells. Although MMAE free payload presented a lower activity compared to cytolysin *in vitro* (Figure 1; Table 1), the corresponding anti-FAP conjugate OMTX105 unexpectedly showed a concentration-dependent killing of HT1080-FAP cells higher than OMTX705 cytolysin conjugate. Here, a concentration of half-maximal killing of 64 pM for OMTX105 was determined compared to 750 pM for OMTX705. This activity was FAP-specific since a strongly reduced activity was observed on HT1080-WT cells for both ADCs, with values of 77,150 pM for OMTX105 and 61,795pM for OMTX705. Specificity ratio between both cell lines was 1,205 for OMTX105 while for OMTX705 the value was 82 (Figure 3).

**Table 4: Comparative in vitro binding and cytotoxic activity values of OMTX105 and OMTX705 ADCs**

| ADC | EC50 values cell binding | | Concentration of half maximal killing | | Specificity ratio between HT1080WT and HT1080FAP |
|---|---|---|---|---|---|
| | HT1080FAP (pM) | HT1080 WT (pM) | HT1080FAP (pM) | HT1080 WT (pM) | |
| OMTX105 | 255.0 | n.d. | 64 ± 33 | 77,150 ± 311 | 1,205 |
| OMTX705 | 203.9 | n.d. | 750 ± 93 | 61,795 ± 4,575 | 82 |

### EXAMPLE 3: Comparative in vivo antitumoral efficacy of MMAE versus cytolysin antiFAP antibody drug conjugates

To evaluate the *in vivo* efficacy of OMTX1 05 and OMTX705, both ADCs were tested in female NOD scid gamma (NSG) mice co-inoculated with HT1080-FAP transfected cells and huPBMCs.

After 7 days, mice were randomized according to tumor size in the different treatment groups. Treatments with OMTX105 and OMTX705 as single agents were initiated, both with a weekly intravenous dose of 20 mg/kg over 3 weeks.

Tumor volume and animal weight were measured 3 times a week to monitor the evolution of tumor growth and weight change upon treatment.

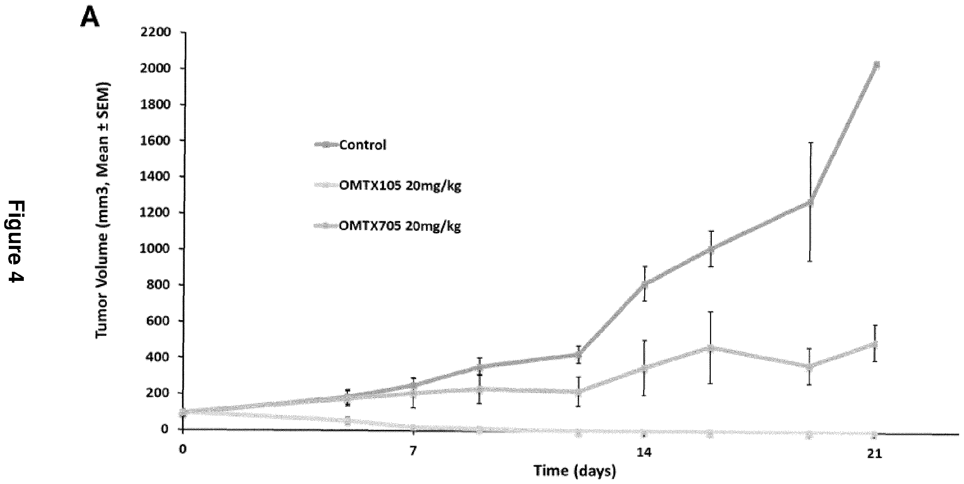

Both OMTX705 and OMTX105 showed high anti-tumor efficacy. OMTX705 showed a percentage tumor growth inhibition (TGI) value of 79.46%, but no complete response after 3 doses. Unexpectedly, OMTX105 showed a greater antitumoral effect, with complete response in this model at day 7, and a percentage TGI of 104.98% (Figure 4A).

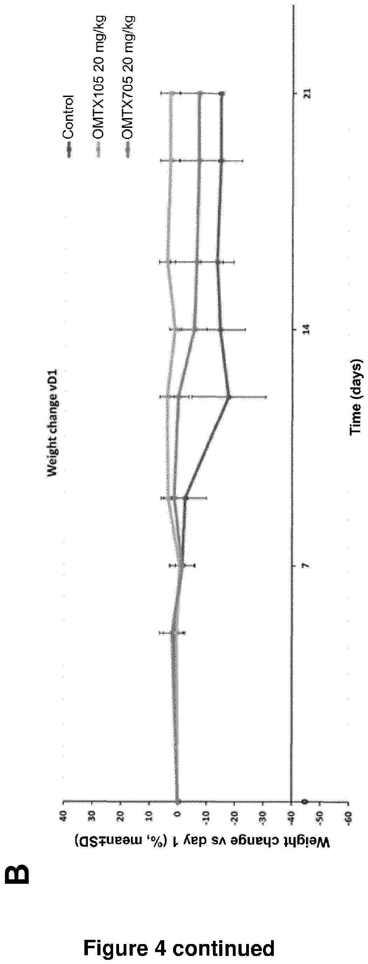

No weight loss, nor toxic effect was observed for OMTX105 and OMTX705 compounds (Figure 4B). Both OMTX105 and OMTX705 were well tolerated at 20 mg/kg, showing less weight loss than the control condition over the 21-day test period.

This experiment showed that contrarily to what was observed *in vitro* for the respective payload activity, an anti-FAP-MMAE ADC shows higher antitumor efficacy than an anti-FAP-cytolisin ADC *in vivo,*

### EXAMPLE 4: Comparative in vivo antitumoral efficacy of MMAE versus cytolysin antiFAP antibody drug conjugates in a patient-derived xenograft model

The antitumor activity of OMTX105 vs OMTX705 was evaluated in the Panc007 pancreatic ductal adenocarcinoma patient derived xenograft mode in female Athymic nude-Foxn1 (nude/nude) mice.

Both OMTX105 and OMTX705 were administered intravenously at 5 and 20 mg/kg once a week for 4 weeks.

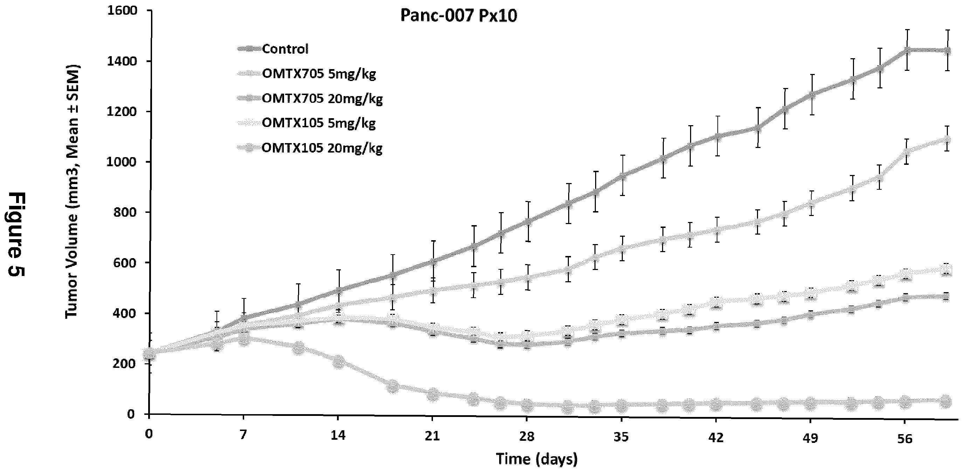

OMTX105 administered at 20 mg/kg once a week showed the highest anti-tumoral efficacy in Panc007 model with a TGI value of 136% on day 28. On the other hand, with the highest dose of OMTX705, 20 mg/kg, the anti-tumoral efficacy was very similar to OMTX105 at the lowest dose of 5 mg/kg with TGI values at day 28 of 92 and 85, respectively (Figure 5).

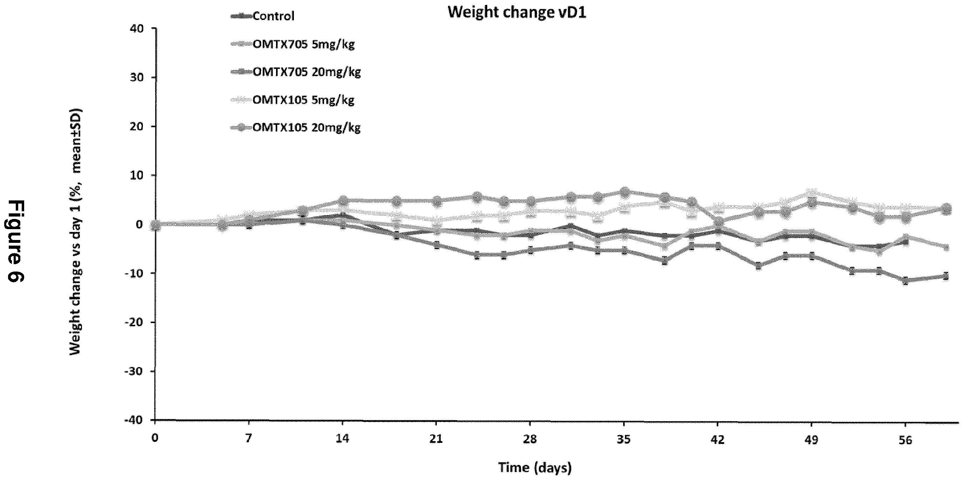

Both OMTX705 and OMTX105 were well tolerated by mice, based on body weight data and clinical observations, with no adverse effects or animal death (Figure 6).

OMTX105 at both 5 mg/kg and 20 mg/kg demonstrated the highest tolerance in mice out of all test conditions, with no weight loss observed after 56 days. This highlights the high tolerance of OMTX105 in mice.

These results showed that, differently to their corresponding free payloads, OMTX105 anti-FAP MMAE ADC has a higher anti-tumoral efficacy than OMTX705 anti-FAP cytolysin conjugate in a pancreatic ductal adenocarcinoma tumor.

### References

A number of publications are cited above in order to more fully describe and disclose the invention and the state of the art to which the invention pertains. Full citations for these references are provided below. The entirety of each of these references is incorporated herein.
For standard molecular biology techniques, see Sambrook, J., Russel, D.W. Molecular Cloning, A Laboratory Manual. 3 ed. 2001, Cold Spring Harbor, New York: Cold Spring Harbor Laboratory Press Shastry, Mythili et al. "Rise of Antibody-Drug Conjugates: The Present and Future." American Society of Clinical Oncology educational book. American Society of Clinical Oncology. Annual Meeting vol. 43 (2023): e390094. doi:10.1200/EDBK_390094
Fu, Zhiwen et al. "Antibody drug conjugate: the "biological missile" for targeted cancer therapy." Signal transduction and targeted therapy vol. 7,1 93. 22 Mar. 2022, doi:10.1038/s41392-022-00947-7
Pettinato MC. Introduction to Antibody-Drug Conjugates. Antibodies (Basel). 2021 Oct 27;10(4):42. doi: 10.3390/antib10040042. PMID: 34842621; PMCID: PMC8628511.
Wang, Zhijia et al. "Antibody-drug conjugates: Recent advances in payloads." Acta pharmaceutica Sinica. B vol. 13,10 (2023): 4025-4059. doi:10.1016/j.apsb.2023.06.015
Samantasinghar, Anupama et al. "A comprehensive review of key factors affecting the efficacy of antibody drug conjugate." Biomedicine & pharmacotherapy = Biomedecine & pharmacotherapie vol. 161 (2023): 114408. Doi:10.1016/j.biopha.2023.114408
Riccardi, Federico et al. "A comprehensive overview on antibody-drug conjugates: from the conceptualization to cancer therapy." Frontiers in pharmacology vol. 14 1274088. 18 Sep. 2023, doi:10.3389/fphar.2023.1274088

## Claims

1. A conjugate having the formula I:
A-(L-D)ₚ (I)
or a pharmaceutically acceptable salt or solvent thereof,
wherein:
A is an antibody comprising
(i) a heavy chain variable (VH) region comprising the following CDRs:
HC-CDR1 having the amino acid sequence of SEQ ID NO:7
HC-CDR2 having the amino acid sequence of SEQ ID NO:8
HC-CDR3 having the amino acid sequence of SEQ ID NO:9
or a variant thereof having one amino acid variation in one or more of HC-CDR1, HC-CDR2, or HC-CDR3; and
(ii) a light chain variable (VL) region comprising the following CDRs:
LC-CDR1 having the amino acid sequence of SEQ ID NO:10
LC-CDR2 having the amino acid sequence of SEQ ID NO:11
LC-CDR3 having the amino acid sequence of SEQ ID NO:12
or a variant thereof having one amino acid variation in one or more of LC-CDR1, LC-CDR2, or LC-CDR3;
L is a linker;
D is a drug comprising a dolastatin; and
p is 1 to 10.

2. The conjugate of claim 1, wherein A comprises:
(i) a heavy chain variable (VH) region comprising the following CDRs:
HC-CDR1 having the amino acid sequence of SEQ ID NO:7
HC-CDR2 having the amino acid sequence of SEQ ID NO:8
HC-CDR3 having the amino acid sequence of SEQ ID NO:9; and
(ii) a light chain variable (VL) region comprising the following CDRs:
LC-CDR1 having the amino acid sequence of SEQ ID NO:10
LC-CDR2 having the amino acid sequence of SEQ ID NO:11
LC-CDR3 having the amino acid sequence of SEQ ID NO:12.

3. The conjugate of claim 1 or claim 2, wherein A comprises a heavy chain variable (VH) region comprising an amino acid sequence having at least 90%, 95%, 99% or 100% sequence identity with SEQ ID NO:5.

4. The conjugate of any one of claims 1 to 3, wherein A comprises a light chain variable (VL) region comprising an amino acid sequence having at least 90%, 95%, 99% or 100% sequence identity with SEQ ID NO:6.

5. The conjugate of any one of claims 1 to 4, wherein A comprises a heavy chain comprising an amino acid sequence having at least 90%, 95%, 99% or 100% sequence identity with SEQ ID NO:3.

6. The conjugate of any one of claims 1 to 5, wherein A comprises a light chain comprising an amino acid sequence having at least 90%, 95%, 99% or 100% sequence identity with SEQ ID NO:4.

7. The conjugate of any one of claims 1 to 6, wherein D is monomethyl auristatin E (MMAE).

8. The conjugate of any one of claims 1 to 7, wherein L comprises a protease-cleavable linker, wherein the linker is cleavable by an intracellular protease.

9. The conjugate of any one of claims 1 to 8, wherein L comprises a valine-citrulline unit, optionally wherein L comprises p-aminobenzylcarbamate, optionally wherein L further comprises maleimidocaproyl-valine-citrulline-p-aminobenzylcarbamate.

10. The conjugate of any one of claims 1 to 9, wherein -L-D has the following structure: 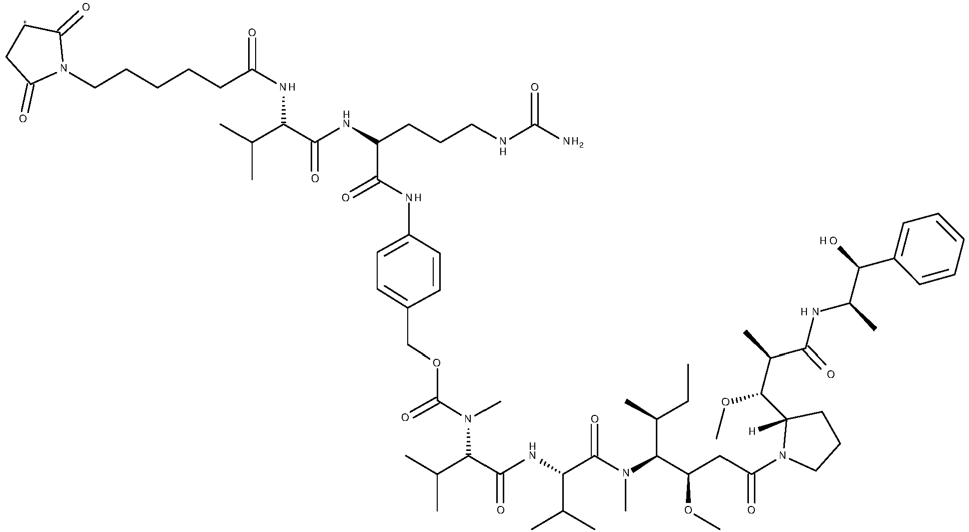 wherein * indicates the site of attachment to A.

11. The conjugate of any one of claims 1 to 10, wherein p is 1, 2, 3, 4 or 5.

12. The conjugate of any one of claims 1 to 11, for use in medicine.

13. The conjugate of any one of claims 1 to 12, for use in a method of treatment of cancer in a mammalian subject.

14. The conjugate for use according to claim 13, wherein the cancer comprises a solid tumor, optionally, wherein the cancer is pancreatic cancer, sarcoma, fibrosarcoma, oesophageal cancer, breast cancer, melanoma, lung cancer, gastric cancer, head and neck cancer, ovarian cancer, bladder cancer or colorectal cancer.

15. The conjugate for use according to claim 13 or claim 14, wherein the method comprises simultaneous, sequential or separate administration of one or more other anti-cancer agents; optionally wherein the one or more anti-cancer agents comprise a cytotoxic chemotherapy agent, an anti-angiogenic agent or an immunotherapeutic agent.
